(19) Europäisches Patentamt
European Patent Office
Office européen des brevets

(11) Veröffentlichungsnummer: **0 498 011 B1**

(12) **EUROPÄISCHE PATENTSCHRIFT**

(45) Veröffentlichungstag der Patentschrift: **26.04.95**

(51) Int. Cl.$^6$: **C07C 229/58**

(21) Anmeldenummer: **91101674.9**

(22) Anmeldetag: **07.02.91**

(54) **Neue Salze der 2-(2,6-Dichloranilino)-phenylessigsäure, Verfahren zu ihrer Herstellung und ihre Verwendung für topisch anwendbare pharmazeutische Zubereitungen.**

(43) Veröffentlichungstag der Anmeldung:
**12.08.92 Patentblatt 92/33**

(45) Bekanntmachung des Hinweises auf die Patenterteilung:
**26.04.95 Patentblatt 95/17**

(84) Benannte Vertragsstaaten:
**AT BE CH DE DK ES FR GB GR IT LI LU NL SE**

(56) Entgegenhaltungen:
**EP-A- 0 245 126**
**DE-A- 3 336 047**
**DE-A- 3 720 896**

(73) Patentinhaber: **HEUMANN PHARMA GMBH & CO**
**Heideloffstrasse 18 - 28**
**D-90478 Nürnberg (DE)**

(72) Erfinder: **Grafe, Ingomar, Dr., Dipl.-Chemiker,**
**Auerbacher Strasse 18,**
**W-8500 Nürnberg 30 (DE)**
Erfinder: **Schickaneder, Helmut, Dr.,**
**Dipl.-Chemiker,**
**Wiesenstrasse 16,**
**W-8501 Eckental (DE)**
Erfinder: **Mörsdorf, Johann Peter, Dr.,**
**Dipl.-Chemiker,**
**Sportplatzstrasse 4,**
**W-8506 Langenzenn (DE)**
Erfinder: **Vergin, Hartmut, Dr.,**
**Dipl.-Biochemiker,**
**Ebenreuther Strasse 32,**
**W-8500 Nürnberg 30, (DE)**
Erfinder: **Ahrens, Kurt-Henning, Dr., Apotheker,**
**Trödelmarkt 42,**
**W-8500 Nürnberg 1 (DE)**

(74) Vertreter: **Kraus, Walter, Dr. et al**
**Patentanwälte Kraus, Weisert & Partner**
**Thomas-Wimmer-Ring 15**
**D-80539 München (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 498 011 B1

## Beschreibung

Die Erfindung betrifft neue Salze der 2-(2,6-Dichloranilino)-phenylessigsäure,ein Verfahren zu ihrer Herstellung und diese Salze enthaltende pharmazeutische Präparate.

Diclofenac ist der INN-Name der Verbindung 2-(2,6-Dichloranilino)-phenylessigsäure, die zur Gruppe der nicht-steroidalen Entzündungshemmer gehört und folgende Formel hat:

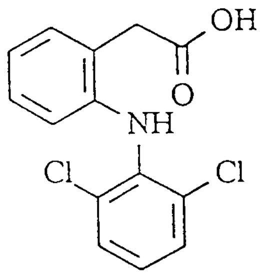

Die Verbindung wird in Form ihres gut in Wasser und Alkoholen löslichen Natriumsalzes bei der Therapie entzündlicher und rheumatischer Prozesse und bei Schmerzzuständen eingesetzt. Sie weist exzellente antiphlogistische und analgetische Wirkungen auf. Bevorzugte galenische Formulierungen sind dabei Tabletten, Dragees, Kapseln, Injektionslösungen und Suppositorien. Da insbesondere bei oraler Anwendung eine Reihe von Nebenwirkungen, wie Magen-Darm-Beschwerden und Störungen der Nieren- und Leberfunktionen, auftreten können, wäre es wünschenswert, topisch, d.h. perkutan anwendbare Zubereitungen von Salzen der 2-(2,6-Dichloranilino)-phenylessigsäure zur Verfügung zu haben, da diese das Zielorgan, z.B. das erkrankte Gelenk, direkt erreichen könnten. Weiterhin würde auch eine erste Metabolisierung in der Leber, die bei oraler Anwendung unvermeidbar ist, ausgeschlossen werden.

In der DE-OS 33 36 047 (GB 2,128,087) werden zwar bereits topisch applizierbare pharmazeutische Zusammensetzungen auf der Basis einer Öl/Wasser-Emulsion mit dem Diethylammoniumsalz der 2-(2,6-Dichloranilino)-phenylessigsäure beschrieben, doch besteht bei Diethylamin die Gefahr der Bildung cancerogener Nitrosamine. Daher hat das Bundesgesundheitsamt im Frühjahr 1987 eine Empfehlung veröffentlicht, in Arzneimitteln und kosmetischen Zubereitungen auf die Verwendung von sekundären Aminen, die zur Nitrosaminbildung neigen, zu verzichten.

Die DE-OS 37 20 896 (GB 2,192,539) beschreibt pharmazeutische Zusammensetzungen, insbesondere für transdermale therapeutische Systeme, die 2-(2,6-Dichloranilino)-phenylessigsäure oder ein pharmazeutisch verwendbares Salz davon, insbesondere das Natrium-, Kalium- oder Diethylammoniumsalz, zusammen mit einer die Permeationsfähigkeit verstärkenden Verbindung enthalten.

Der vorliegenden Erfindung liegt daher die Aufgabe zugrunde, neue Salze der 2-(2,6-Dichloranilino)-phenylessigsäure mit verbesserten Eigenschaften und topisch anwendbare pharmazeutische Präparate mit verbesserten Penetrationseigenschaften bereitzustellen.

Diese Aufgabe wird erfindungsgemäß durch neue Salze der 2-(2,6-Dichloranilino)-phenylessigsäure der allgemeinen Formel (I)

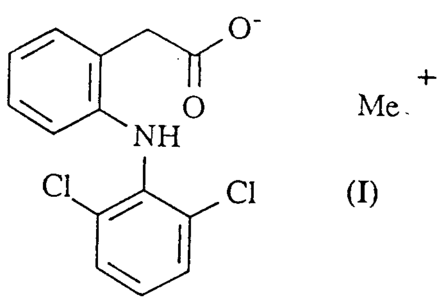

(I)

in der Me für ein Rubidium- oder Caesiumatom steht, gelöst.

Die erfindungsgemäßen Alkalimetallsalze der 2-(2,6-Dichloranilino)-phenylessigsäure sind bislang noch nicht in der Literatur beschrieben. Sie stellen hochschmelzende stabile Festkörper dar, die sich durch eine

unerwartet hohe Lipophilie auszeichnen, d.h. sie haben einen verhältnismäßig geringen salzartigen Charakter. So lösen sich beide nur sehr mäßig in Wasser, aber gut in unpolaren, organischen Lösungsmitteln, z.B. Ketonen wie Methylisobutylketon. Das Rubidium- und das Caesiumsalz zeichnen sich gegenüber allen beschriebenen Salzen der 2-(2,6-Dichloranilino)-phenylessigsäure durch eine überraschende und unerwartet hohe Permeationsfähigkeit durch die Haut aus.

Die erfindungsgemäßen Salze werden durch ein Verfahren hergestellt, das dadurch gekennzeichnet ist, daß man 2-(2,6-Dichloranilino)-phenylessigsäure oder das Natriumsalz davon mit einem geeigneten Rubidium- oder Caesiumsalz in wäßrigalkoholischer Lösung oder in einem wäßrig-organischen Zweiphasensystem umsetzt.

Geeignete Rubidium- oder Caesiumsalze sind die Chloride, Bromide, Sulfate oder Carbonate, wobei die Carbonate bevorzugt werden.

Die Umsetzung der Rubidium- bzw. Caesiumsalze mit der 2-(2,6-Dichloranilino)-phenylessigsäure oder dem Natriumsalz davon kann einerseits in einem homogenen Medium aus Wasser und einem mit Wasser mischbaren niederen Alkohol, beispielsweise Methanol, Ethanol oder Isopropanol, erfolgen. Andererseits ist es auch möglich, in einem Zweiphasensystem aus einer wäßrigen und einer organischen Phase zu arbeiten. Geeignete organische Lösungsmittel hierfür sind Ether, wie tert. Butylmethylether oder Diethylether, Ester, wie z.B. Ethylacetat, oder Ketone, beispielsweise Methylethylketon oder Methylisobutylketon, wobei Ketone bevorzugt werden. Die Reaktionspartner werden vorzugsweise im Molverhältnis 1:1 und bei Temperaturen zwischen 0 und 80 °C, vorzugsweise bei Raumtemperatur, umgesetzt.

Die Isolierung und Reinigung der neuen Salze der 2-(2,6-Dichloranilino)-phenylessigsäure erfolgt nach üblichen Methoden, z.B. Extraktion, Fällung und/oder Kristallisation aus geeigneten Lösungsmitteln.

Schließlich wird durch die Erfindung noch ein pharmazeutisches Präparat zur Verfügung gestellt, das dadurch gekennzeichnet ist, daß es als Wirkstoff ein wie oben definiertes Salz der 2-(2,6-Dichloranilino)-phenylessigsäure, zusammen mit einem physiologisch annehmbaren, für die topische Anwendung geeigneten organischen Lösungsmittel und gegebenenfalls weitere physiologisch annehmbare Hilfsstoffe enthält.

Wie bereits oben zum Ausdruck gebracht, zeichnen sich die erfindungsgemäßen Salze gegenüber den bekannten Salzen der 2-(2,6-Dichloranilino)-phenylessigsäure durch eine überraschende und unerwartet hohe Permeationsfähigkeit durch die Haut aus. Diese überraschenden Eigenschaften wurden in dem nachfolgend beschriebenen Modell der in vitro-Hautpermeation an Hairless-Ratten nachgewiesen. Dazu wurden 3,14 $cm^2$ große Stücke Haut aus dem thorakal-abdominalen Bereich von Hairless-Ratten in einer Penetrationskammer nach T.J. Franz (J. Invest. Dermatol. 64, 190 (1975)) montiert.

Die zu bestimmenden Salze der 2-(2,6-Dichloranilino)-phenylessigsäure wurden in Ethanol/Isopropanol 1:1 (v/v) gelöst, wobei die Einwaage der Salze auf 10,0 mg/ml 2-(2,6-Dichloranilino)-phenylessigsäure-Natrium bezogen wurde. Jeweils 1 ml der Lösungen wurde auf die Hautoberfläche aufgetragen.

Die Akzeptorflüssigkeit bestand aus ca. 8 ml 1/15 M Sörensen Phosphatpuffer, pH 7,4. Nach jeweils 8 Stunden Permeationsdauer wurden die Gesamtmengen der 2-(2,6-Dichloranilino)-phenylessigsäure in der gesamten Akzeptorflüssigkeit mittels HPLC bestimmt.

Ergebnisse:

| Salz der 2-(2,6-Dichloranilino)-phenylessigsäure | Zahl der Einzelversuche | Gesamtmenge 2-(2,6-Dichloranilino)-phenylessigsäure (μg) |
|---|---|---|
| Natrium- | 10 | 11,2 ± 7,4 |
| Kalium- | 19 | 19,0 ± 7,7 |
| Diethylammonium- | 5 | 5,2 ± 1,4 |
| Rubidium- | 5 | 52,1 ± 9,8 |
| Caesium- | 5 | 44,1 ± 10,9 |
| VERGLEICH: freie Säure | 6 | 1,6 ± 0,3 |

Die topische, perkutane Applikation von Arzneimitteln kann in Form von Cremes, Salben, Gelen oder Pasten erfolgen.

Zur Herstellung dieser Arzneimittelformen werden die üblichen Lösungsmittel und Hilfsstoffe verwendet. Als ölige Phasen für Cremes werden z.B. Fettalkohole, Fettsäuren, partielle Fettsäureester des Glycerins oder natürliche oder halbsynthetische Fette verwendet. Für die Salben können geeignete, bei Körpertemperatur flüssige Paraffine oder Vaselinöl als ölige Phasen dienen. Die wäßrigen Phasen von Cremes und Salben können weiterhin Polyalkohole, wie Glycerin oder Propylenglykol, enthalten.

Als Lösungsmittel in Gelen können Dimethylsulfoxid, niedere Alkohole, wie Ethanol oder Isopropanol, und Glykole verwendet werden.

Gemäß einer bevorzugten Ausführungsform der Erfindung enthalten die erfindungsgemäßen pharmazeutischen Präparate eine die Permeation verstärkende Verbindung. Beispiele hierfür sind die Amide höherer Fettsäuren, wie N,N-Dimethyllauroylamid. Als gelbildende Materialien eignen sich anorganische und organische Makromoleküle. Anorganische gelbildende Verbindungen sind z.B. Silikate, insbesondere Aluminium- und Magnesium-Aluminiumsilikate, und kolloidale Kieselsäuren. Aus dem Gebiet der organischen Makromoleküle können natürliche, halbsynthetische oder synthetische Polymere als Gelbildner Verwendung finden. Beispiele für die ersten beiden Gruppen sind z.B. Polysaccharide und deren Derivate, wie Stärke, Gelatine, Agar-Agar, Alginate oder Carboxymethylcellulose. Geeignete gelbildende, synthetische Makromoleküle sind Polymere auf der Basis von Vinylalkoholen, Vinylpyrrolidon, Acrylsäure- und Methacrylsäurederivaten.

Weitere Hilfsstoffe für die genannten Arzneimittelformen sind Emulgatoren, wie nichtionische oder anionische Tenside, Konservierungsmittel, Riechstoffe usw.

Bei den oben angeführten Hilfs- bzw. Trägerstoffen handelt es sich um die üblichen Substanzen, die bei der Herstellung solcher Arzneimittelformen verwendet werden. Auch die Herstellung der pharmazeutischen Präparate erfolgt nach den in der Galenik bekannten Methoden. So können z.B. die genannten Komponenten und Hilfsstoffe in den vorgesehenen Mengen vermischt und die erhaltene Mischung in an sich bekannter Art und Weise zu den topischen Formulierungen verarbeitet werden.

Die auf diese Weise erhaltenen, topisch anwendbaren pharmazeutischen Präparate können zur Behandlung von Entzündungen und Schmerzzuständen, insbesondere bei rheumatischen Erkrankungen in der Human- und Veterinärmedizin eingesetzt werden. Solche Erkrankungen sind z.B. entzündlich-rheumatische Gelenk- und Wirbelsäulenleiden einschließlich Gichtanfällen, Reizzustände bei degenerativen Gelenk- und Wirbelsäulenleiden, Weichteilrheumatismus und schmerzhafte Schwellungen oder Entzündungen nach Verletzungen oder Operationen, wobei insbesondere bei der Behandlung von entzündlichen oder entzündlich aktivierten degenerativen Formen des Rheumatismus gute Ergebnisse erwartet werden können.
Die folgenden Beispiele erläutern die Erfindung:

Beispiel 1

2-(2,6-Dichloranilino)-phenylessigsäure-Rubidiumsalz

Zu einer Lösung von 17,3 g (75 mmol) Rubidiumcarbonat in 180 ml Ethanol und 30 ml Wasser werden 44,3 g (150 mmol) 2-(2,6-Dichloranilino)-phenylessigsäure portionsweise zugegeben.

Nach kurzem Rühren wird die Lösung im Vakuum eingedampft, durch azeotrope Destillation mit 50 ml Methylisobutylketon entwässert und abermals im Vakuum zur Trockene eingedampft.

Nach Umkristallisation des Rückstandes aus Isopropanol erhält man 52,0 g (91% der Theorie) grobe, farblose Prismen, die bei 90 °C Kristallwasser verlieren und bei 270 °C schmelzen.
$C_{14}H_{10}Cl_2NO_2Rb$ (380,62)

Beispiel 2

2-(2,6-Dichloranilino)-phenylessigsäure-Caesiumsalz

Analog Beispiel 1 werden 19,61 g (60 mmol) Caesiumcarbonat und 35,4 g (120 mmol) 2-(2,6-Dichloranilino)-phenylessigsäure umgesetzt.

Nach Umkristallieren aus Methylisobutylketon werden 51,0 g (99% der Theorie) farblose Schuppen vom Schmelpunkt 245 °C erhalten.
$C_{14}H_{10}ClCsNO_2$ (428,05)

## Patentansprüche

**Patentansprüche für folgende Vertragsstaaten : AT, BE, CH, DE, DK, FR, GB, IT, LI, LU, NL, SE**

1. Salze der 2-(2,6-Dichloranilino)-phenylessigsäure der allgemeinen Formel (I)

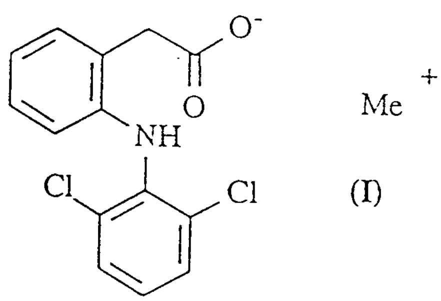

(I)

in der Me für ein Rubidium- oder Caesiumatom steht.

2. Salz nach Anspruch 1, dadurch **gekennzeichnet,** daß in der allgemeinen Formel (I) Me für ein Rubidiumatom steht.

3. Salz nach Anspruch 1, dadurch **gekennzeichnet,** daß in der allgemeinen Formel (I) Me für ein Caesiumatom steht.

4. Verfahren zur Herstellung der Salze nach den Ansprüchen 1 bis 3, dadurch **gekennzeichnet,** daß man 2-(2,6-Dichloranilino)-phenylessigsäure oder das Natriumsalz davon mit einem geeigneten Rubidium- oder Caesiumsalz in wäßrig-alkoholischer Lösung oder in einem wäßrig-organischen Zweiphasensystem umsetzt.

5. Verfahren nach Anspruch 4, dadurch **gekennzeichnet,** daß man als Rubidium- oder Caesiumsalze die Chloride, Bromide, Sulfate oder Carbonate verwendet.

6. Pharmazeutisches Präparat, dadurch **gekennzeichnet,** daß es als Wirkstoff ein Salz nach einem der Ansprüche 1 bis 3, zusammen mit einem physiologisch annehmbaren, für die topische Anwendung geeigneten organischen Lösungsmittel und gegebenenfalls weitere physiologisch annehmbare Hilfsstoffe enthält.

7. Pharmazeutisches Präparat nach Anspruch 6, dadurch **gekennzeichnet,** daß es das für die topische Anwendung geeignete organische Lösungsmittel im Gemisch mit Wasser enthält.

8. Pharmazeutisches Präparat nach Anspruch 6 oder 7, dadurch **gekennzeichnet,** daß es weiterhin eine die Permeation verstärkende Verbindung enthält.

**Patentansprüche für folgende Vertragsstaaten : ES, GR**

1. Verfahren zur Herstellung der Salze der 2-(2,6-Dichloranilino)-phenylessigsäure der allgemeinen Formel (I)

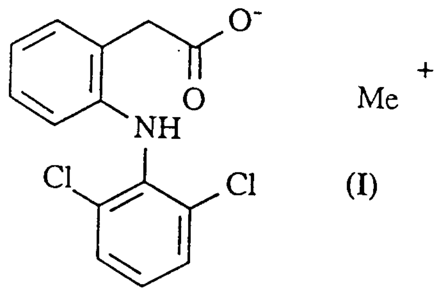

(I)

in der Me für ein Rubidium- oder Caesiumatom steht, dadurch **gekennzeichnet,** daß man 2-(2,6-Dichloranilino)-phenylessigsäureoder das Natriumsalz davon mit einem geeigneten Rubidium- oder Caesiumsalz in wäßrig-alkoholischer Lösung oder in einem wäßrig-organischen Zweiphasensystem umsetzt.

2. Verfahren nach Anspruch 1, dadurch **gekennzeichnet,** daß man als Rubidium- oder Caesiumsalze die Chloride, Bromide, Sulfate oder Carbonate verwendet.

**Claims**
**Claims for the following Contracting States : AT, BE, CH, DE, DK, FR, GB, IT, LI, LU, NL, SE**

1. Salts of 2-(2,6-dichloroanilino)phenylacetic acid of the general formula (I)

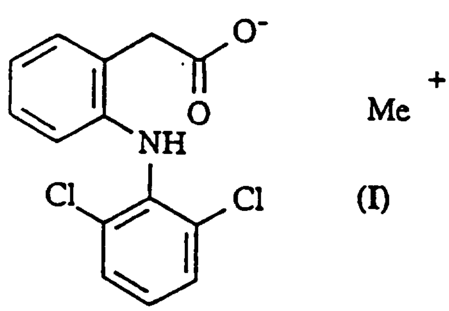

(I)

in which Me denotes a rubidium or caesium atom.

2. Salt according to claim 1, characterised in that Me in the general formula (I) denotes a rubidium atom.

3. Salt according to claim 1, characterised in that Me in the general formula (I) denotes a caesium atom.

4. Process for the production of the salts according to the claims 1 to 3, characterised in that 2-(2,6-dichloroanilino)phenylacetic acid or the sodium salt thereof is reacted with a suitable rubidium or caesium salt in an aqueous-alcoholic solution or in an aqueous-organic two-phase system.

5. Process according to claim 4, characterised in that the rubidium or caesium salts used are their chlorides, bromides, sulphates or carbonates.

6. Pharmaceutical preparation, characterised in that it contains as active ingredient a salt according to one of claims 1 to 3, together with a physiologically compatible organic solvent suitable for topical application and optionally further physiologically compatible auxiliary substances.

7. Pharmaceutical preparation according to claim 6, characterised in that it contains the organic solvent suitable for topical application as a mixture with water.

8. Pharmaceutical preparation according claim 6 or 7, characterised in that it additionally contains a compound which promotes permeation.

**Claims for the following Contracting States : ES, GR**

1. Process for the production of the salts of 2-(2,6-dichloroanilino)phenylacetic acid of the general formula (I)

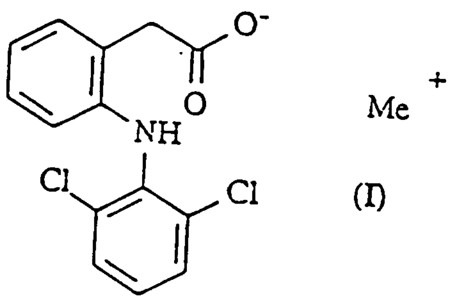

(I)

in which Me denotes a rubidium or caesium atom, characterised in that 2-(2,6-dichloroanilino)-phenylacetic acid or the sodium salt thereof is reacted with a suitable rubidium or caesium salt in an aqueous-alcoholic solution or in an aqueous-organic two-phase system.

2. Process according to claim 1, characterised in that the rubidium or caesium salts used are their chlorides, bromides, sulphates or carbonates.

**Revendications**
**Revendications pour les Etats contractants suivants : AT, BE, CH, DE, DK, FR, GB, IT, LI, LU, NL, SE**

1. Sels de l'acide 2-(2,6-dichloranilino)phénylacétique de formule générale (I) :

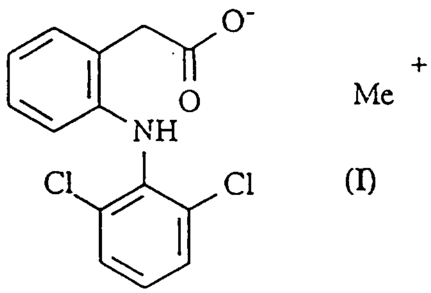

(I)

dans laquelle Me représente un atome de rubidium ou de césium.

2. Sel selon la revendication 1, caractérisé en ce que, dans la formule générale (I), Me représente un atome de rubidium.

3. Sel selon la revendication 1, caractérisé en ce que, dans la formule générale (I), Me représente un atome de césium.

4. Procédé de préparation des sels selon les revendications 1 à 3, caractérisé en ce que l'on fait réagir l'acide 2-(2,6-dichloranilino)phénylacétique ou son sel de sodium avec un sel de rubidium ou de césium approprié dans une solution hydroalcoolique ou dans un système à deux phases aqueux-organique.

5. Procédé selon la revendication 4, caractérisé en ce que l'on utilise comme sels de rubidium ou de césium les chlorures, bromures, sulfates ou carbonates.

6. Préparation pharmaceutique caractérisée en ce qu'elle contient comme principe actif un sel selon l'une des revendications 1 à 3 ainsi qu'un solvant organique physiologiquement acceptable, convenant pour l'utilisation topique, et éventuellement d'autres adjuvants physiologiquement acceptables.

7. Préparation pharmaceutique selon la revendication 6, caractérisée en ce quelle contient le solvant organique convenant pour l'utilisation topique en mélange avec de l'eau.

8. Préparation pharmaceutique selon la revendication 6 ou 7, caractérisée en ce qu'elle contient en outre un composé renforçant la perméation.

**Revendications pour les Etats contractants suivants : ES, GR**

1. Procédé de préparation des sels de l'acide 2-(2,6-dichloranilino)phénylacétique de formule générale (I) :

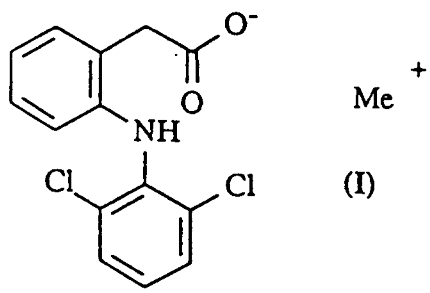

(I)

dans laquelle Me représente un atome de rubidium ou de césium, caractérisé en ce que l'on fait réagir l'acide 2-(2,6-dichloranilino)phénylacétique ou son sel de sodium avec un sel de rubidium ou de césium approprié dans une solution hydroalcoolique ou dans un système à deux phases aqueux-organique.

2. Procédé selon la revendication 1, caractérisé en ce que l'on utilise comme sels de rubidium ou de césium les chlorures, bromures, sulfates ou carbonates.